# EUROPEAN PATENT APPLICATION

(11) **EP 2 289 391 A1**
(43) Date of publication of application: **02.03.2011**
(21) Application number: 10174151.0
(22) Date of filing: 26.08.2010
(51) Int. Cl.: A61B 1/227

(54) **Medical inspection device**

(30) Priority: 26.08.2009 US 547673; 08.10.2009 US 575469
(71) Applicant: Apple Biomedical, Inc., Zhongshan Dist., Taipei (TW)
(72) Inventor: Huang, Tzai-Kun, Taipei (TW)
(74) Representative: 2K Patentanwälte Blasberg Kewitz & Reichel

(57) **Abstract**

An improved medical inspection device comprises a hand-held module, a display module, coupled to the hand-held module, a first circuit module electrically connected to the display module, and an inspection module. The display module includes an image display module, settled on a first surface of the display module, for displaying a video signal received by the display module. The inspection module is detachably coupled to the display module and comprises an inspection portion arranged at a front end of the inspection module. The inspection portion comprises a lens for taping an image of an external target object, an image sensing unit for receiving the image taken by the lens and converting the image into a video signal, a light source for emitting and projecting a light onto the target object, and a second circuit module configured to be capable of being electrically connected to the first circuit module and transmit the video signal from the image sensing unit to the display module.

## Description

### CROSS REFERENCE TO RELTED APPLICATION

This application claims priority of a co-pending U.S. Patent Application Ser. No. 12/575,469, filed Oct. 8, 2009 by the present inventor, the whole content of which is hereby incorporated by reference. This application also claims priority from a co-pending U.S. Patent Application Ser. No. 12/547,673 filed Aug. 26, 2009, the whole content of which is hereby incorporated by reference.

### BACKGROUND OF THE INVENTION

### 1. Technical Field

The present invention relates to a medical inspection device, and more particularly, to an improved medical inspection device including a hand-held portion, a display portion, and a detachable inspection module.

### 2. Description of Related Art

It is common that doctors use conventional hand-held examining devices for otologic, peroral or nasal endoscopy to facilitate diagnosing patients. Such a conventional hand-held examining device is configured like a magnifying glass, having a grip shank and an observing part. The observing part has its front end formed tapered for easily entering an organ to be examined. At the distal end of the tapered part, there is a lens for magnifying an image of the interior of organs so as to facilitate a doctor's diagnosis.

However, in use of the conventional hand-held examining device, to observe the magnified image, the user (i.e. the doctor) has to approach his/her eye to the observing part to look steadily at the lens. This can probably bring eyestrain to the user, and thus is not an ideal way to make observations. Besides, in use of the conventional hand-held examining device, additional light sources are usually required to illuminate the dark interior of organs for clear observation, thus resulting in another inconvenience. Moreover, after observation is made by using such a hand-held examining device, the only way to record the observed images is the doctor's sketches on paper. Consequently, the correctness of anamneses can be deteriorated if any inaccuracy exists in such sketches.

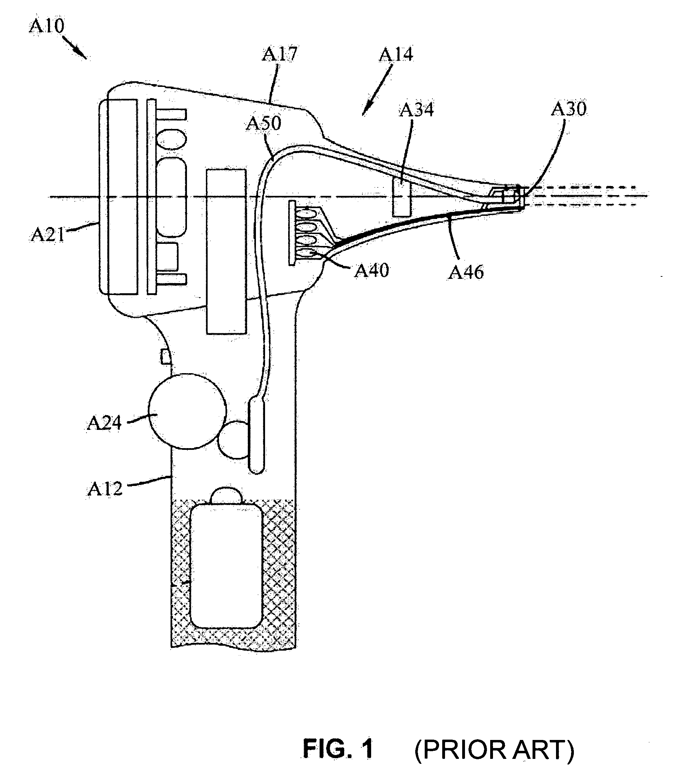

In view of this, there has been proposed a medical inspection device for remedying the shortcomings of the traditional hand-held examining devices. FIG. 1 is a sectional view of a conventional medical inspection device. The medical inspection device A10 includes a body A12 for a user to grip, a display portion A17 that has a screen A21, a speculum portion A14 that has a lens A30 at a front end thereof for entering an organ to be examined and capturing images, a sensor A34 converting the images into video signals; a focus control switch A24 for adjusting a focal length of the lens A30 through a cable A50, and a plurality of light generator A40, which are LEDs for emitting light beams that are then projected to an external target object through a light pipe A46 for illuminating the field of view. Therein, the body A12, the display portion A17, and the speculum portion A14 are enclosed by an integrally formed housing and made not detachable.

After every single time of use, the speculum portion A14 directly contacting a patient's organ needs to be disinfected. However, since the medical inspection device A10 is integratedly formed, it is impossible to detach the speculum portion A14 for separate disinfection, thus being very inconvenient. In another case where any of the components inside the speculum portion A14 fails, replacement or repair of the component in problem can never be accomplished unless the entire medical inspection device A10 is disassemble.

On the other hand, along the route defined by the light pipe A46 where the light emitted by the light generator A40 proceeds to the target object, the optical energy is more or less lost during the transmission along the light pipe A46. Besides, the light pipe A46 itself increases the overall manufacturing cost.

Moreover, such medical device can be used for only one purpose, in this case, for examining the interior of an ear. If the user desires to examine other organs, for example, eyes, nose, skin lesion or internal organs, separate devices, such as an ophthalmascope, rhinoscope, dermatoscope or endoscope, will have to be obtained. The cost for obtaining all the devices may be very high.

In view of this, it is necessary to provide an improved medical inspection device that addresses all the shortcomings of the prior art devices.

### SUMMARY OF THE INVENTION

Hence, to remedy the problems and disadvantages of the prior art, the inventor of the present invention, relying on his years of experience, fully implemented his imagination and creativeness, to repeatedly experiment and make modification, and eventually developed an improved medical inspection device as claimed in the present application.

Examples of the present invention provide a medical inspection device, which comprises a body and at least one inspection module, where each of the at least one inspection module has a configuration, different from one another, suitable for entering the cavity of a particular organ. At any given time, one of the at least one inspection module can be detachably coupled to the body of the medical inspection device, so as to allow more convenient repair and maintenance of the medical inspection device and a wider variety of applications of the medical inspection device.

Some examples of the present invention also provide a medical inspection device, which has a light source settled at an end of its speculum portion, so as to save the use of any additional light pipe that is required in the prior art devices, thereby reducing the manufacturing cost.

Therefore, the present invention provides an improved medical inspection device, which at least comprises a hand-held module, a display module, coupled to the hand-held module, a first circuit module electrically connected to the display module, and an inspection module. The display module comprising an image display module, settled on a first surface of the display module, for displaying a video signal received by the display module. The inspection module detachably coupled to the display module. The inspection module may be configured to be applicable to one of dermatoscope, nasoscope, ophthalmoscope, octoscope, and endoscope. The inspection module comprising a lens for taking an image of an external target object, an image sensing unit for receiving the image taken by the lens and converting the image into a video signal, a light source for emitting and projecting a light onto the target object, and a second circuit module configured to be capable of being electrically connected to the first circuit module and transmit the video signal from the image sensing unit to the display module.

### BRIEF DESCRIPTION OF THE DRAWINGS

The invention as well as preferred modes of use, further objectives and advantages thereof will be best understood by reference to the following detailed description of illustrative embodiments when read in conjunction with the accompanying drawings, wherein:

FIG. 1 is a sectional view of a conventional medical inspection device;

FIG. 2 is a perspective view of a medical inspection device according to a first preferred embodiment of the present invention;

FIG. 3 is a partially exploded view of the medical inspection device according to the first preferred embodiment of the present invention;

FIG. 4 is a partially exploded view of the medical inspection device according to the first preferred embodiment of the present invention taken from a different viewpoint;

FIG. 5 is a sectional view of the medical inspection device according to the first preferred embodiment of the present invention;

FIG. 6 is an enlarged drawing showing the circled area of FIG. 5;

FIG. 7 is a schematic diagram illustrating electrical transmission among partial components of the medical inspection device according to the first preferred embodiment of the present invention;

FIG. 8 is a perspective view of a medical inspection device according to a second preferred embodiment of the present invention;

FIG. 9 is a sectional view of the medical inspection device according to the second preferred embodiment of the present invention;

FIG. 10 is a schematic diagram illustrating electrical transmission among partial components of the medical inspection device according to the second preferred embodiment of the present invention;

FIG. 11 is a partially exploded view of a medical inspection device according to a third preferred embodiment of the present invention;

FIG. 12 is a partially exploded view of a medical inspection device according to a fourth preferred embodiment of the present invention;

FIG. 13 is a partially exploded view of a medical inspection device according to a fifth preferred embodiment of the present invention; and

FIG. 14 is a partially exploded view of a medical inspection device according to a sixth preferred embodiment of the present invention.

### DETAILED DESCRIPTION OF THE PREFERRED EMBODIMENTS

To achieve the foregoing objectives and effects, the inventor of the present invention makes improvement to the conventional medical inspection device, by configuring the inspection module as a unit detachable from other components of the medical inspection device, and rearranging the internal components, so as to excogitate an improved medical inspection device of the present invention. Hereinafter, six preferred embodiments of the medical inspection device of the present invention will be described in detail so as to illustrate the structural and technical features of the present invention.

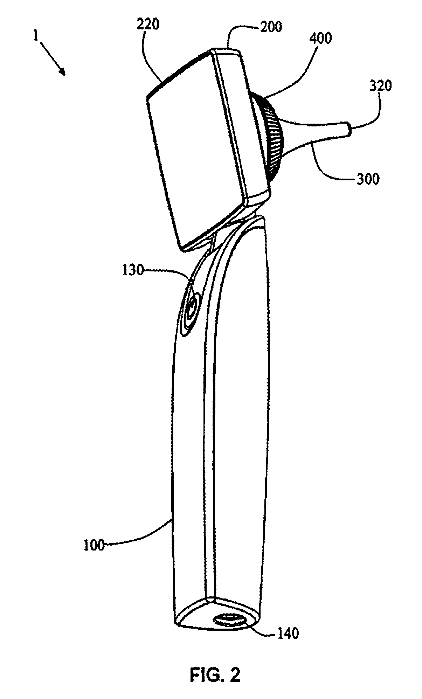

FIGs. 2-6 show a medical inspection device according to a first preferred embodiment of the present invention. FIG. 2 is a perspective view of a medical inspection device according to a first preferred embodiment of the present invention. The first preferred embodiment of the present invention primarily comprises a hand-held portion 100, a display portion 200, an inspection module 300, and a fastening ring 400.

The hand-held portion 100 is provided for a user to grip, thereby facilitating the user's operation of the medical inspection device 1.

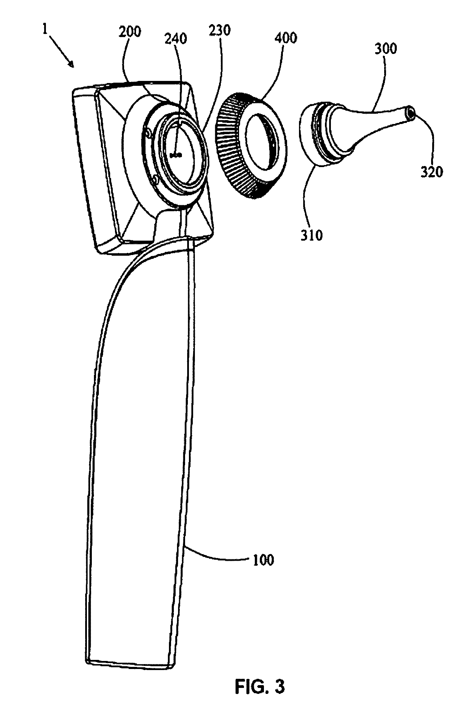

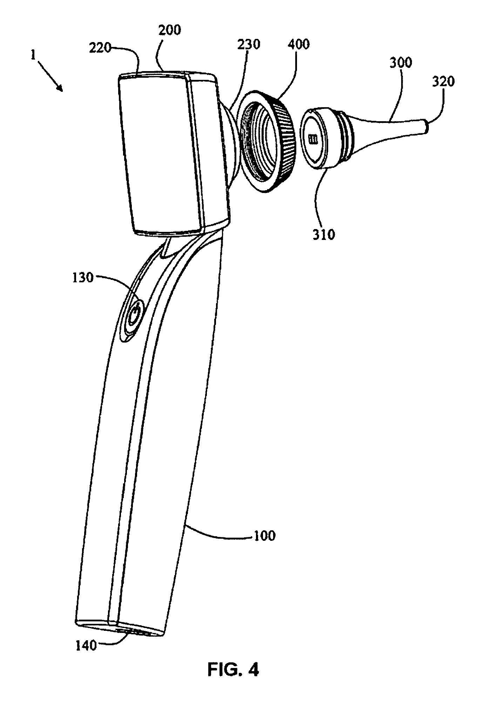

FIG. 3 is a partially exploded view of the medical inspection device according to the first preferred embodiment of the present invention. FIG. 4 is a partially exploded view of the medical inspection device according to the first preferred embodiment of the present invention from a viewpoint different from that of FIG. 3.

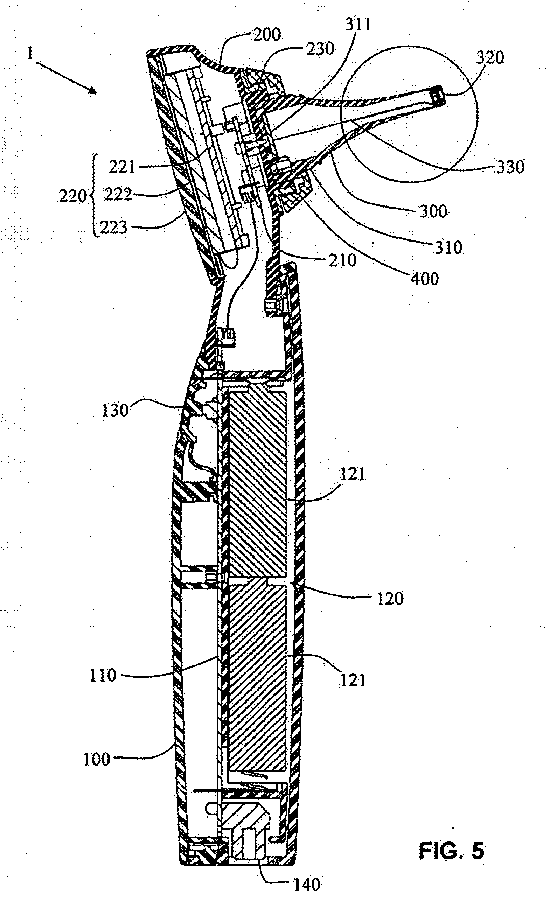

FIG. 5 is a sectional view of the medical inspection device according to the first preferred embodiment of the present invention. The display portion 200 is fixedly coupled with one end of the hand-held portion 100. The display portion 200 further comprises a first printing circuit board 210 settled inside the display portion 200, and an image display module 220 settled on a surface of the display portion 200 for displaying a particular image. The image display module 220 is composed of a fourth printing circuit board 221, a screen 222, and a transparent protective cover 223. The fourth printing circuit board 221 is electrically connected with the first printing circuit board 210 for transmitting power and signals. The screen 222 abuts on a surface of the fourth printing circuit board 221 opposite to a surface of the fourth printing circuit board 221 connected with the first printing circuit board 210. The transparent protective cover 223 abuts on a surface of the screen 222 opposite to a surface of the screen 222 abutting on the fourth printing circuit board 221. Therein, the screen 222 is a liquid crystal display (LCD). An annular protuberance 230 is settled on surface of the display portion 200 opposite to the surface of the display portion 200 having the image display module 220. In addition, a plurality of electrode contacts 240 are settled on the surface of the display portion 200 in an area circled by the annular protuberance 230 and are electrically connected with the first printing circuit board 210.

The inspection module 300 may be detachably assembled with the hand-held portion 100, the display portion 200 and the fastening ring 400 to form an otoscope. The inspection module 300 further comprises a joint portion 310 and an inspection portion 320. The joint portion 310 is settled at a rear end of the inspection module 300 and the inspection portion 320 is arranged at a front end of the inspection module 300. The joint portion 310 has a second printing circuit board 311 arranged on its inner surface. The joint portion 310 is designed to be fittingly engaged in a space bordered by the annular protuberance 230 of the display portion 200. The plurality electrode contacts 240 are configured to contact the second printing circuit board 311 so as to cause the first printing circuit board 210 and the second printing circuit board 311 electrically connected with each other.

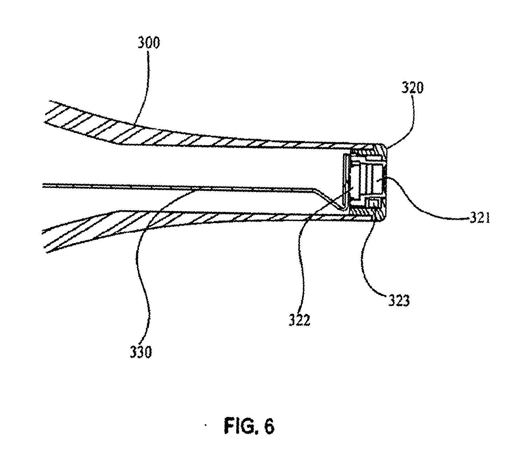

FIG. 6 is an enlarged drawing showing the circled area of FIG. 5. The inspection module 300 is configured for inserting into the ear cavity for examination of the internal cavity of the ear. For example, the inspection module 300 may have a wide diameter at a rear end that tapers inwardly to a narrower diameter at a front end. The inspection portion 320 is located at the front end of the inspection module 300 and comprises a lens 321, an image sensing unit 322, and a light source 323. The lens 321 is settled and exposed at a front end of the inspection portion 320 for taking an image of an external target object. The image sensing unit 322 is settled inside the inspection portion 320 and connected with the lens 321 for receiving the image taken by the lens 321 and converting the image into a video signal. The light source 323 is settled inside the inspection portion 320 and adjacent to the lens 321, and serves to emit and project a light onto the target object for improving clearness of the taken image. Therein, the light source 323 is a light emitting diode (LED). However, in practice, the light source may alternatively be a miniature incandescent bulb, a compact fluorescent light (CFL) or a laser device. A third printing circuit board 330 is settled inside the inspection module 300 and connected between the image sensing unit 322 and the second printing circuit board 311. The image sensing unit 322 thus is able to transmit the video signal of the image to the image display module 220 by way of the third printing circuit board 330, the second printing circuit board 311, and the first printing circuit board 210, and to display the image through the screen 222. Therein, the third printing circuit board 330 is a flexible printed circuit (FPC).

The fastening ring 400 may be detachably coupled to the annular protuberance 230 of the display portion 200, so as to make the joint portion 310 of the inspection module 300 firmly coupled to the display portion 200.

In the foregoing first preferred embodiment, matching screw threads are further provided at an outer periphery of the annular protuberance 230 and an inner periphery of the fastening ring 400, respectively, so that by tightening the screw threads of the outer and inner peripheries together, the annular protuberance 230 and the fastening ring 400 can be firmly fastened together. However, in practical application, the annular protuberance 230 and the fastening ring 400 may be alternatively fastened by means of wedging or other screw fasteners. Moreover, the display portion 200 may be directly assembled with the inspection module 300 by means of wedging, without using the fastening ring 400.

Furthermore, in the first preferred embodiment, the hand-held portion 100 further comprises components described below. A fifth printing circuit board 110 is settled inside the hand-held portion 100 and electrically connected with the first printing circuit board 210 in the display portion 200 for controlling power and transmitting video signals. An accommodating space 120 serves to accommodate two batteries 121. The two batteries 121 are electrically connected with the fifth printing circuit board 110 for providing power required by the medical inspection device 1. A power button switch 130 is electrically connected with the fifth printing circuit board 110 for turning on or off the power supply. An image transmission port 140 is electrically connected with the fifth printing circuit board 110 for transmitting the image taken by the lens 321 to an external device through a transmission line.

In the first preferred embodiment of the present invention, in addition to the image transmission port 140, a radio frequency (RF) module (not shown) may be further provided so as to wirelessly transmit image data to an external device through radio frequency. The RF module may be optionally settled inside the hand-held portion 100 or the display portion 200.

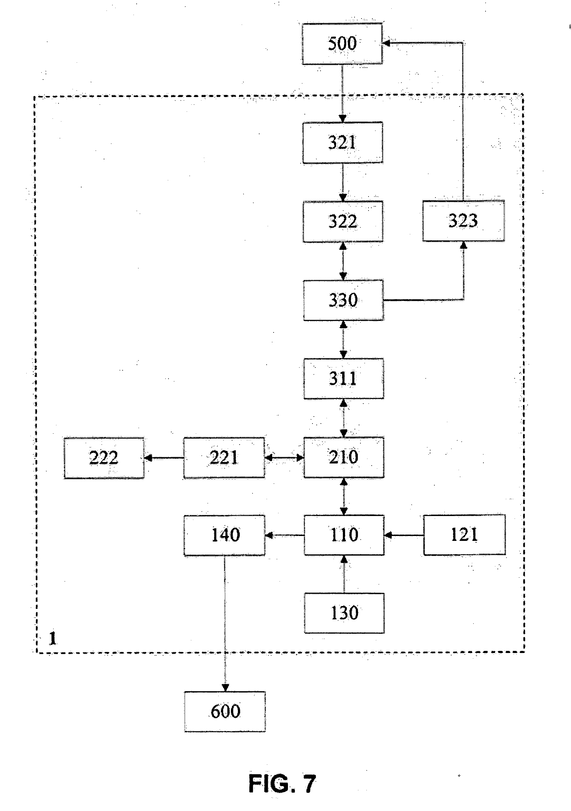

Please refer to FIG. 7 for a schematic diagram illustrating electrical transmission among partial components of the medical inspection device according to the first preferred embodiment of the present invention. As can be seen in the diagram, the medical inspection device 1 is powered by the batteries 121. The power supply is transmitted to the second printing circuit board 311, the third printing circuit board 330, and the fourth printing circuit board 221 through the fifth printing circuit board 110 and the first printing circuit board 210, respectively. Then the third printing circuit board 330 delivers the power to the image sensing unit 322, the lens 321 and the light source 323 for their use while the fourth printing circuit board 221 delivers the power to the screen 222 for its use. Furthermore, the light source 323 projects the light onto a target object 500, and the lens 321 captures an image of the target object 500. Afterward, the image is converted into a video signal by the image sensing unit 322. The video signal may be subsequently transmitted to the screen 222 for display or transmitted to the image transmission port 140 so as to be further transmitted to an external device 600 through a transmission line. In addition, the power button switch 130 may be operated to turn on or off the power supply through the fifth printing circuit board 110.

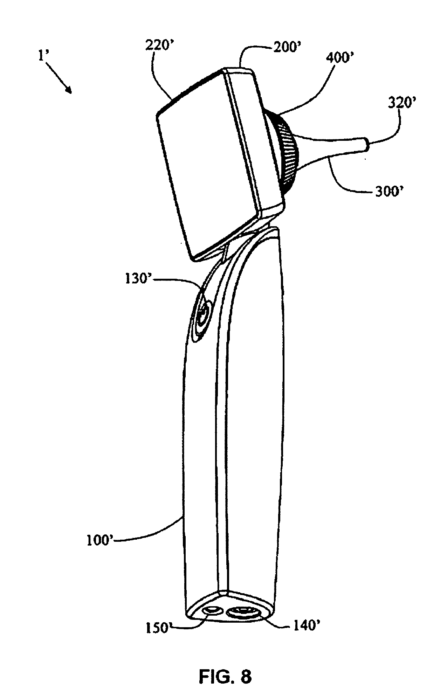

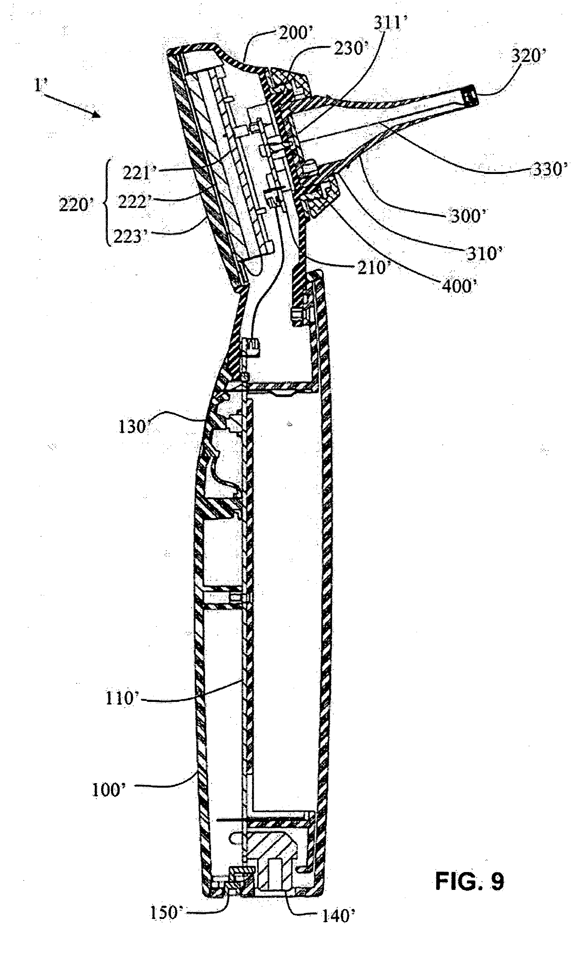

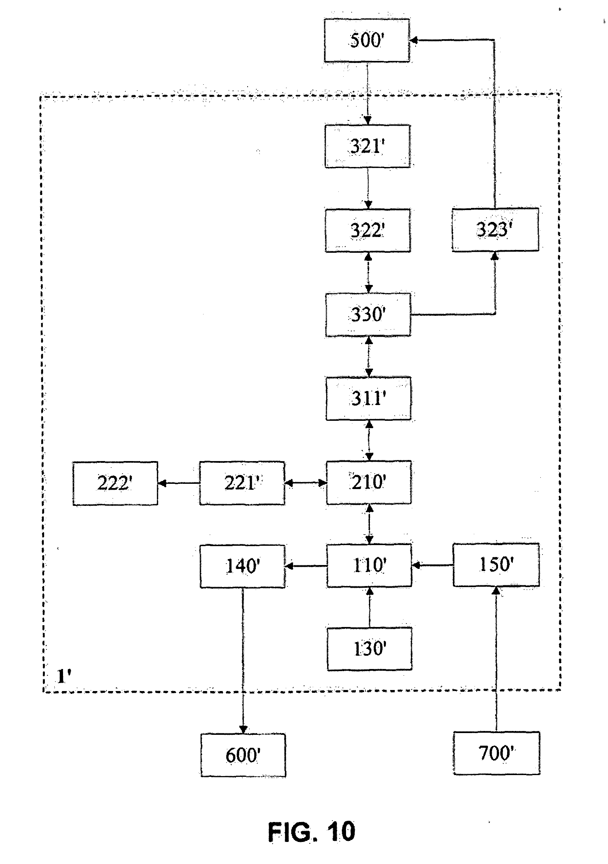

FIGs. 8 to 10 show a medical inspection device according to a second preferred embodiment of the present invention. FIG. 8 is a perspective view of the medical inspection device according to the second preferred embodiment of the present invention. FIG. 9 is a sectional view of the medical inspection device according to the second preferred embodiment of the present invention. FIG. 10 is a schematic diagram illustrating electrical transmission among partial components of the medical inspection device according to the second preferred embodiment. While the second preferred embodiment has most components in common with those of the first preferred embodiment, the details of these previously described comments are omitted herein. The difference between the two embodiments relies on that in a medical inspection device 1' according to the second preferred embodiment, a hand-held portion 100' has a power port 150', which is electrically connected with a fifth printing circuit board 110' settled inside the hand-held portion 100'. The power port 150' is designed to connect an external power source 700', which provides the power required by the medical inspection device 1'.

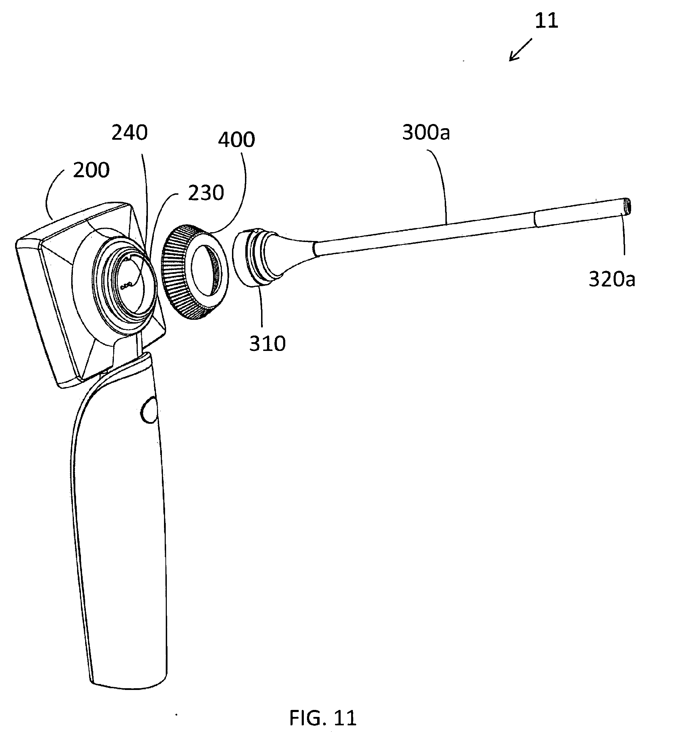

FIG. 11 is a partially exploded view of a medical inspection device 11 according to a third preferred embodiment of the present invention. Referring to FIG. 11, the third preferred embodiment of the present invention may be similar to the first and second embodiments of the present invention except that the inspection module 300a may be detachably assembled with the hand-held portion 100, the display portion 200 and the fastening ring 400 to form a rhinoscope. Specifically, the inspection module 300a is configured so as to allow it to be inserted into the nasal cavity for examination inside the nose. For example, the inspection module 300a may have a wide diameter at a rear end that tapers inwardly to a narrower diameter and further extends to a front end to form an inspection portion 320a.

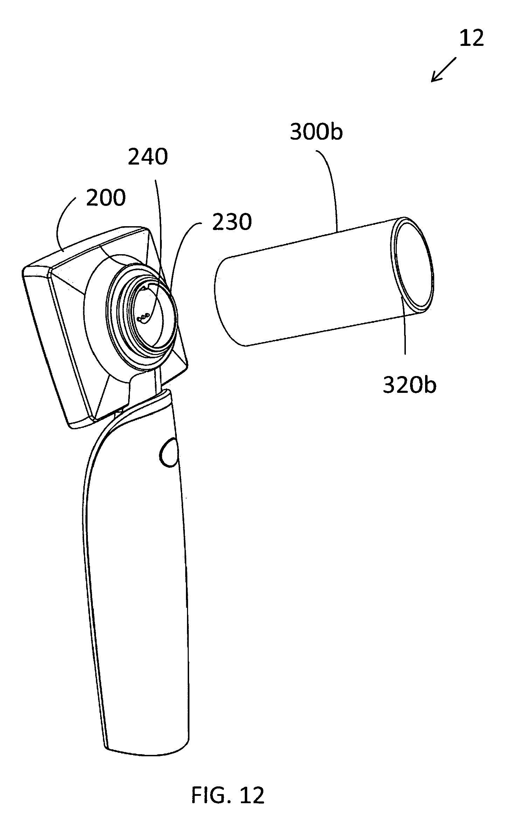

FIG. 12 is a partially exploded view of a medical inspection device 12 according to a fourth preferred embodiment of the present invention. Referring to FIG. 12, the fourth preferred embodiment of the present invention may be similar to the first and second embodiments of the present invention except that the inspection module 300b may be detachably assembled with the hand-held portion and display portion 200 to form an ophthalmoscope. Specifically, the inspection module 300b is configured for examination of the eye. For example, inspection module 300b may have the form of a cylinder with the inspection portion 320b located at a front end of the inspection module 300b.

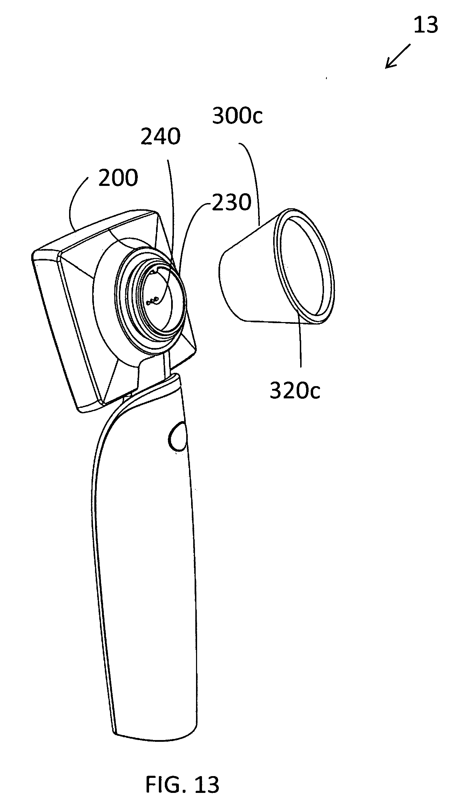

FIG. 13 is a partially exploded view of a medical inspection device 13 according to a fifth preferred embodiment of the present invention. Referring to FIG. 13, the fifth preferred embodiment of the present invention may be similar to the first and second embodiments of the present invention except that the inspection module 300c may be detachably assembled with the hand-held portion 100 and the display portion 200 to form a dermatoscope. Specifically, the inspection module 300c is configured for the examination of skin condition, such as skin lesion. For example, the inspection module 300 may have the form of a cone with a narrow diameter at a rear end and a inspection portion 320c at a front end.

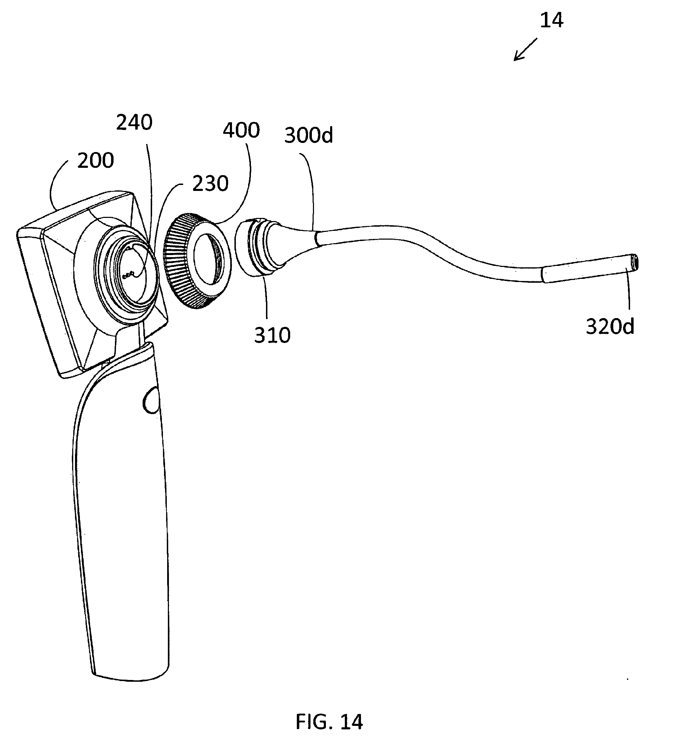

FIG. 14 is a partially exploded view of a medical inspection device 14 according to a sixth preferred embodiment of the present invention. Referring to FIG. 14, the sixth preferred embodiment of the present invention may be similar to the first and second embodiments of the present invention except that the inspection module 300d may be detachably assembled with the hand-held portion 100, the display portion 200, and the fastening ring 400 to form a larygoscope, laparoscope, cytoscope, arthroscope or veterinary endoscope. Specifically, the inspection module 300d is configured for examination of, for example, joint, interior of abdominal or pelvic cavity, bladder, urethra, vocal folds, glottis, lungs or airway passage. For example, the inspection module 300d may comprise a joint portion 310 having a wide diameter rear end that tapers inwardly to a narrower diameter and an inspection portion 320d being a flexible tube extending out from the joint portion 310. The diameter and length of the flexible tube may vary depending on the intended application.

Through the above detailed illustration of the overall structural and technical features of the present invention, it can be summarized that the present invention has at least the following advantages:
1. The inspection module of the present invention is detachably assembled with the display portion, so that when the inspection module fails, when an inspection module with different functions is needed, or when the inspection module has to be disinfected independently, the currently assembled inspection module can be easily detached for repair, replacement or disinfection, thereby making the medical inspection device more convenient in uses and maintenance.
2. The present invention provides a plurality of inspection modules, each having a configuration, different from another, suitable for entering the cavity of a particular organ, and each can be detachably assembled with other components of the medical inspection device, so that when examining different organs, the user can select the appropriate inspection module, depending on the need of the user, to be coupled with the other components of the medical inspection device.
3. The present invention uses the fastening ring to firmly fasten the inspection module to the display portion, so as to prevent the inspection module from coming off of the display portion, thus improving structural stability in use.
4. In the present invention, the light source is directly settled at the front end of the inspection module, so as to give immediate illumination to the target object in inspection and save the use of any light pipe that is required in the prior art devices, thus reducing the manufacturing cost.
5. The present invention converts the image of the target object into the video signal by using the image sensing unit arranged at the front end of the inspection portion and displays the image through the screen, thereby allowing easy observation of the image.

## Claims

1. A medical inspection device comprising:
a hand-held module (100);
a display module (200), coupled to the hand-held module, comprising an image display module (220), settled on a first surface of the display module, for displaying a video signal received by the display module;
a first circuit module (210) electrically connected to the display module; and
an inspection module (300) detachably coupled to the display module, the inspection module comprising an inspection portion (320) arranged at a front end of the inspection module, wherein the inspection portion comprises:
a lens (321) for taking an image of an external target object,
an image sensing unit (322) for receiving the image taken by the lens and converting the image into a video signal,
a light source (323) for emitting and projecting a light onto the target object, and
a second circuit module (311) configured to be capable of being electrically connected to the first circuit module (210) and transmit the video signal from the image sensing unit to the display module.

2. The medical inspection device of claim 1, wherein the inspection module (300) is configured to have a rear end having a first diameter
a) that tapers inwardly to the front end having a second diameter; or
b) that tapers inwardly to a second diameter, and further extends to the front end forming the inspection portion.

3. The medical inspection device of claim 1, wherein the inspection module (300) is configured to have a form of a cylinder or to have a form of a cone with a rear end having a first diameter and the front end having a second diameter, the second diameter being greater than the first diameter.

4. The medical inspection device of claim 1, wherein the inspection module further comprises a first portion configured to have a rear end having a first diameter that tapers inwardly to a second diameter, and a second portion of a flexible tube extending out from the first portion, and the inspection portion is located at a front end of the flexible tube.

5. The medical inspection device of any of the preceding claims, wherein the display module (200) further comprises:
an annular protuberance (230), settled on another surface of the display module (200) opposite to the surface having the image display module (220), and
at least one electrode contact (240), settled on the surface of the display module (200) in an area circled by the annular protuberance, and electrically connected with the first circuit module (210).

6. The medical inspection device of claim 5, wherein the inspection module further comprises:
a joint portion (310) having a shape configured to engage with the annular protuberance (230) such that when the joint portion is engaged with the annular protuberance, the second circuit module (311) is electrically connected to the at least one electrode contact (240).

7. The medical inspection device of claim 6, further comprising:
a fastening ring (400), detachably fastened to the annular protuberance (230) of the display module, so as to firmly affix the joint portion of the inspection module to the display module.

8. The medical inspection device of claim 7, wherein matching screw threads are further provided at an outer periphery of the annular protuberance and an inner periphery of the fastening ring, respectively, so that by tightening the screw threads of the inner and outer peripheries together, the annular protuberance and the fastening ring are firmly fastened together.

9. The medical inspection device of claim 7, wherein the annular protuberance (230) and the fastening ring (400) are fastened together by means of wedging or by screw fasteners.

10. The medical inspection device of any of the preceding claims, wherein the light source is selected from the group consisting of a light emitting diode (LED), a miniature incandescent bulb, a compact fluorescent light (CFL), and a laser device.

11. The medical inspection device of any of the preceding claims, wherein the first circuit module is further configured to transmit power and data to the display module.

12. The medical inspection device of any of the preceding claims, wherein the hand-held module comprises
an accommodating space for accommodating at least one battery that is electrically connected to the first circuit module for providing power required by the medical inspection device; or
a power port that is electrically connected to the first circuit module, the power port configured to connect with an external power source for providing power required by the medical inspection device.

13. The medical inspection device of claim 12 or 13, wherein the hand-held module comprises a power button switch electrically connected to the first circuit module for turning on or off the power.

14. The medical inspection device of any of the preceding claims, further comprising
an image transmission port electrically connected to the first circuit module for transmitting the image taken by the lens to an external device through a transmission line; or
a radio frequency (RF) module for wirelessly transmitting the image data to an external device through radio frequency.

15. The medical inspection device of any of the preceding claims, wherein the image sensing unit is selected from the group consisting of a complementary metal oxide semiconductor (CMOS), a charge coupled device (CCD), and an image system-on-a-chip (image SOC).
